# EUROPÄISCHE PATENTSCHRIFT

(11) **EP 3 164 095 B1**
(45) Veröffentlichungstag und Bekanntmachung des Hinweises auf die Patenterteilung: **13.03.2019**
(21) Anmeldenummer: 15729480.2
(22) Anmeldetag: 17.06.2015
(51) Int. Cl.: A61C 3/04, A61C 19/02

(54) **LAGERUNGSSYSTEM FÜR MEDIZINISCHE INSTRUMENTE**
STORAGE SYSTEM FOR MEDICAL INSTRUMENTS
SYSTÈME DE STOCKAGE D'INSTRUMENTS MÉDICAUX

(30) Priorität: 01.07.2014 DE 102014109197
(43) Veröffentlichungstag der Anmeldung: 10.05.2017
(60) Teilanmeldung: 18204465.1
(73) Patentinhaber: Aesculap AG, 78532 Tuttlingen (DE)
(72) Erfinder: ZIERIS, Gerold, 78570 Mühlheim (DE); WEISSHAUPT, Dieter, 78194 Immendingen (DE); BELLIKLI, Serkan, 72355 Schömberg (DE)
(74) Vertreter: Winter, Brandl, Fürniss, Hübner, Röss, Kaiser, Polte - Partnerschaft mbB
(86) Internationale Anmeldenummer: PCT/EP2015/063576
(87) Internationale Veröffentlichungsnummer: WO 2016/000962

(56) Entgegenhaltungen:
- WO-A2-2005/053597
- US-A- 5 918 740
- US-A1- 2008 230 423
- US-A1- 2012 094 249
- US-A1- 2013 046 289
- US-A1- 2013 064 709
- US-A1- 2014 170 592

## Beschreibung

Die vorliegende Erfindung betrifft ein Lagerungssystem für medizinische Instrumente insbesondere Dentalinstrumente gemäß dem Oberbegriff des Patentanspruchs 1.

### Hintergrund der Erfindung

In der modernen Chirurgie kommt eine Vielzahl von verschiedenen Instrumentenköpfen/Werkzeugen wie z.B. Bohr- oder Fräsköpfen zum Einsatz, die jeweils auf/in ein einen Instrumentenantrieb umfassendes Handstück abnehmbar auf-/eingesteckt werden können. Dies erlaubt dem Operateur, während des Betriebs des Handstücks den auf dem / in das Handstück auf-/eingesteckten Instrumentenkopf je nach Anforderung der Anwendung zu wechseln. Um dem Operateur hierbei einen möglichst einfachen Zugriff auf diese Instrumentenköpfe (nachstehend als Werkzeuge bezeichnet) zu ermöglichen, werden die Werkzeuge gewöhnlich in einem chirurgischen Werkzeugset geordnet aufbewahrt. Hierbei werden, um Beschädigungen der Werkzeuge zu vermeiden und ein rasches Auffinden eines benötigten Werkzeugs zu gewährleisten, die Werkzeuge nicht lose aufbewahrt, sondern in Aufnahmehülsen aufgenommen, die in einer Lochmatrix des Werkzeugsets eingesteckt bzw. darin fixiert sind. Um dem Operateur ein Auffinden des jeweils benötigten Werkzeugs zu erleichtern, sind die Aufnahmehülsen oft farbkodiert. Zudem kann eine Schablone mit aufgedruckten OP-Pfaden auf die Aufnahmehülsen aufgelegt werden, um die Auswahl des jeweils erforderlichen Werkzeugs weiter zu vereinfachen.

### Stand der Technik

Herkömmliche Werkzeugsets für die Lagerung/Aufbewahrung von chirurgischen Werkzeugen sind von verschiedenen Anbietern beziehbar und gehören damit zum allgemeinen Stand der Technik. Diese bekannten Werkzeugsets bestehen im Wesentlichen aus einem Kunststoff- oder Metallkasten mit einem auf- und zuklappbaren Deckel, in den parallel zum Boden des Kastens eine Zwischenetage oder Einlage zwischen Boden und Deckel des Kastens eingesetzt ist. Die Zwischenetage weist runde Löcher auf, in die (Kunststoff-) Aufnahmehülsen für die Aufnahme von Werkzeugen eingesetzt sind. Die Aufnahmehülsen sind hierbei als einstückige, massive Hohlzylinder ausgebildet, die jeweils eine Aufnahmeöffnung für ein bestimmtes chirurgisches Werkzeug aufweisen. Chirurgische Werkzeuge insbesondere Dentalwerkzeuge oder Drehwerkzeuge dieser Gattung haben einen distalen Werkzeugeingriffsabschnitt und einen proximalen Schaftabschnitt, wobei Aufnahmehülsen mit verschiedenen Durchmessern der Werkezugaufnahmeöffnungen die korrekte und sitzgenaue Aufnahme von Werkzeugen mit verschiedenen Schaftdurchmessern erlauben. Weiterhin sind die Aufnahmehülsen farbkodiert für ein einfacheres Auffinden eines benötigten Werkzeugs durch einen Operateur, wobei vorzugsweise eine bedruckte Schablone vorgesehen sit, die über die Aufnahmehülsen gelegt werden kann und mit OP-Pfaden und Bezeichnungen der jeweils in einer der Aufnahmehülsen aufgenommen Werkzeuge bedruckt ist.

US 2013/0064709 A1 offenbart eine Aufnahmehülse eines Werkzeugsets gemäß dem Oberbegriff des Anspruchs 1.

Neben der schonenden Lagerung chirurgischer Werkzeuge und deren raschen Auffindbarkeit während einer Operation, ist die gründliche und häufige Reinigung und ggf. Sterilisation chirurgischer Werkzeuge von größter Wichtigkeit.
Während sich herkömmlichen Werkzeugsets gut für eine Lagerung chirurgischer Werkzeuge eignen, und auch ein rasches Auffinden des jeweils benötigten Werkzeugs während einer Operation gewährleisten, besteht ein Nachteil dieser herkömmlichen Werkzeugsets darin, dass chirurgische Werkzeuge zur Reinigung aus einem herkömmlichen Werkzeugset (Aufbewahrungskasten) entnommen und danach wieder in dieses eingeordnet werden müssen, da eine Reinigung der Werkzeuge, während diese in dem Instrumentenset (Aufbewahrungskasten) eingesetzt sind, bei herkömmlichen Instrumentensets nicht oder nur schwer möglich ist. Dies liegt vor Allem an dem massiven Aufbau der Aufnahmehülsen und des Instrumentensets an sich, der eine ausreichende Umströmung der darin aufbewahrten Werkzeuge mit Reinigungsflüssigkeiten oder -gasen weitestgehend verhindert.
Reinigungsfluide dringen nur schwer in den Kunststoffkasten des Werkzeugsets ein und durch den massiven Aufbau der Aufnahmehülsen und deren große Kontaktfläche mit den aufgenommenen Instrumenten werden die in den Aufnahmehülsen aufgenommen Anteile der Werkzeuge nur unzureichend gereinigt.

Da bis zu 90 verschiedene chirurgische Werkzeuge in einem einzigen Werkzeugset aufgenommen sein können, ist ein Herausnehmen aller Werkzeuge zur Reinigung und ein nachfolgendes Wiederbestücken des Werkzeugsets sehr zeitaufwändig. Zudem müssen chirurgische Werkzeuge für eine längere Lebensdauer schonend behandelt werden, um zum Beispiel ein Abstumpfen/Beschädigen von Spitzen oder Kanten zu verhindern, und nach jeder Anwendung gründlich gereinigt werden, was den Zeitaufwand weiter vergrößert.

Zusätzlich lagern sich Verschmutzungen auch an dem Werkzeugset selbst an, wenn dieses wiederholt zur Aufnahme verschmutzter Werkzeuge z. B. während einer Operation verwendet wird. Wird ein verschmutztes Werkzeug in eine Aufnahmehülse zurückgeführt, so können an dem Werkzeug klebende Verschmutzungen wie Knochensplitter oder Blut in dem Hohlzylinder der Aufnahmehülse haften bleiben oder auf die auf den Aufnahmehülsen aufgelegte Schablone fallen. Auch muss der Raum zwischen dem Boden des Werkzeugsets und der Zwischenetage, in die die Aufnahmehülsen eingesetzt sind, sehr sauber gehalten werden. Daher müssen herkömmliche Werkzeugsets zur Reinigung der Aufnahmehülsen und der übrigen Bestandteile des Sets (Kunststoffkasten, Deckel, Zwischenetage) zerlegt werden und nachfolgend die verschiedenen Bestandteile des Werkzeugsets einzeln gereinigt und das Set danach wieder zusammengesetzt werden. Auch hier ist der Zeitaufwand sehr groß und die vielen Schritte des Reinigungsverfahrens (Zerlegen in Einzelteile, Reinigung jedes einzelnen Teils) bieten viele Gelegenheiten für Beschädigungen des Werkzeugsets oder Anwenderfehler bei der Reinigung. Da durch den massiven Aufbau der Aufnahmehülsen Reinigungsflüssigkeiten nur schwer in die Aufnahmeöffnung der Aufnahmehülsen eindringen können, muss gegebenenfalls eine manuelle Nachreinigung der Aufnahmeöffnungen durchgeführt werden.

Um hohen Temperaturen bei der Sterilisation widerstehen zu können, müssen Kunststoffanteile herkömmlicher Werkzeugsets aus speziellem temperaturresistentem Kunststoff gefertigt sein, was die Fertigungskosten erhöht. Zudem bleiben Verschmutzungen an Kunststoff aufgrund dessen positiver Oberflächenladung oft länger haften als an anderen Werkstoffen wie beispielsweise Metall, was die Reinigung des Werkzeugsets zusätzlich erschwert. Auch bei der Trocknung des Werkzeugsets nach der Reinigung erweist sich der massive Aufbau herkömmlicher Werkzeugsets und deren Fertigungsmaterial Kunststoff als nachteilig.

Aus dem Stand der Technik sind daher Werkzeugsets und Aufnahmehülsen für chirurgische Instrumente bekannt, die aufgrund des massiven Aufbaus der Aufnahmehülsen und Werkzeugsets eine Reinigung der aufgenommenen chirurgischen Instrumente in bestücktem Zustand des Werkzeugsets (Aufbewahrungskasten) nicht ermöglichen.

### Kurzbeschreibung der Erfindung

Ausgehend von dem vorstehend beschriebenen Stand der Technik liegt der Erfindung zu Aufgabe zugrunde, eine Aufnahmehülse, sowie eine Halterungsvorrichtung für diese Aufnahmehülse und ein chirurgisches Instrumentenset zu schaffen, die eine effiziente und vereinfachte Reinigung des Werkzeugsets, vorzugsweise in bestücktem Zustand, sowie der darin enthaltenen chirurgischen Instrumente, ermöglichen.

Diese Aufgabe wird gelöst durch eine Aufnahmehülse gemäß Anspruch 1 und ein chirurgisches Werkzeugset gemäß Anspruch 12. Vorteilhafte Weiterbildungen sind hierbei Gegenstand der Unteransprüche.

Ein Grundgedanke der Erfindung besteht im Wesentlichen darin, dass sowohl die Aufnahmehülse als auch das gesamte Werkzeugset (bzw. Aufnahmekasten mit Hülsen-Halterungsvorrichtung und Aufnahmehülse) durch eine filigrane Bauweise möglichst durchlässig für Reinigungs- bzw. Trocknungsfluid wird, damit möglichst viel Reinigungs- bzw. Trocknungsfluid an die im Werkzeugset aufgenommen Werkzeuge gelangen kann. Durch eine damit vereinfachte Reinigung chirurgischer Werkzeuge und ganzer Werkzeugsets werden zudem der für die Aufbereitung dieser benötigte Zeitaufwand und die Bereithaltungskosten dieser gesenkt. Weiterhin können die Herstellungs- und Anschaffungskosten von Aufnahmehülsen für chirurgische Werkzeuge und von Werkzeugsets gesenkt werden.

Ein weiterer Grundgedanke der Erfindung besteht im Wesentlich darin, die im Werkzeugset eingesetzte Aufnahmehülsen mit einer Art innerer Werkzeughalterung zu versehen, welche das eingesteckte Werkzeug im Wesentlichen punkt- oder linienförmig hält, sodass zwischen eingestecktem Werkzeug und Aufnahmehülse (radiale) Freiräume verbleiben, die zum großflächigen Umströmen des eingesteckten Werkzeugs mit Reinigungsfluid nutzbar sind.

Der Kern der vorliegenden Erfindung besteht demzufolge darin, die Aufnahmehülse, sowie die Bestandteile der die Aufnahmehülse stützenden Halterungsvorrichtung und des die Halterungsvorrichtung aufnehmenden Werkzeugsets (Kasten), in welche die Aufnahmehülse eingesetzt ist, sowie ggf. etwaige zusätzliche weitere Teile des erfindungsgemäßen Werkzeugsets (in Filigran-/Gerüstbauweise) als durchbrochene Körper von lichtem Aufbau auszubilden, um so eine umfängliche Durchlässigkeit für Reinigungsfluide zu gewährleisten und nur geringe Kontaktflächen mit dem Werkzeug zu erzeugen. Dieser lichte Aufbau steht im Gegensatz zu dem massiven Aufbau der aus dem Stand der Technik bekannten Aufnahmehülsen, Halterungsvorrichtungen für Aufnahmehülsen und Werkzeugsets (einschließlich Kasten, Halterungsvorrichtung und Aufnahmehülsen) und erlaubt es, das Werkzeug in der Aufnahmehülse sowie im Werkzeugset (Werkzeugkasten) zu belassen, wenn dieses einem Reinigungs-/Sterilisationsvorgang zugeführt werden soll. Damit bleibt das Werkzeug gegen äußere Einwirkungen im Rahmen eines Reinigungsvorgangs geschützt. Gleichzeitig wird nicht nur das Werkzeug sondern auch die Aufnahmehülse und das gesamte Werkzeugset mit gereinigt/sterilisiert.

Unter einem lichten Aufbau wird im Sinne dieser Anmeldung ein Aufbau eines Körpers/Kastens verstanden, bei dem der Körper/Kasten möglichst viele Aussparungen aufweist und möglichst viel durchbrochen ist. Unter massivem Aufbau wird im Sinne dieser Anmeldung hingegen ein Aufbau eines Körpers/Kastens verstanden, bei dem der Körper/Kasten wenige bis keine Aussparungen aufweist und daher wenig bis gar nicht durchbrochen ist. In anderen Worten ausgedrückt definiert sich der Begriff "lichter Aufbau" vorzugsweise darin, dass die Gesamtfläche der Durchbrüche/Aussparrungen in der Aufnahmehülse/im Kasten größer ist als die vom Hülsen-/Kastenmaterial abgedeckte Gesamtfläche.

Erfindungsgemäß besteht die Aufnahmehülse demnach aus einem Körper von lichtem Aufbau (Gerüstbauweise) gemäß vorstehender Definition in Form eines Hohlzylinders/Hohlkörpers mit einer axialen Werkzeug-Aufnahme-/Einstecköffnung an einem ersten axialen Ende, in die chirurgische Werkzeuge eingeführt werden können, wobei der Hohlzylinder/Hohlkörper der Aufnahmehülse dazu ausgelegt ist, die Kontaktfläche zwischen Aufnahmehülse und einem darin aufgenommen Werkzeug zu minimieren und einen maximalen Fluidfluss (radial) durch den Hohlkörper der Aufnahmehülse und an das in der Aufnahmehülse aufgenommene Werkzeug zu ermöglichen. Vorzugsweise ist die Aufnahmehülse hierbei einstückig z. B. im Spritzgussverfahren oder nach dem Rapid-Prototyping-Verfahren (Sinterverfahren) aus einem Kunststoff gefertigt und zeichnet sich durch eine möglichst ebenflächige Oberfläche ohne Spalte oder Hinterschnitte aus, um einer Ablagerung von Verschmutzungen entgegenzuwirken.

In einer Ausführungsform weist die Aufnahmehülse an ihrer Innenseite eine Anzahl elastisch verformbarer Vorsprünge (bzw. Klemmarme/Haltekrallen) auf, die innerhalb des Hohlzylinders der Aufnahmehülse vorzugsweise einen Trichter bilden, der sich in Einführungsrichtung eines aufzunehmenden Werkzeugs verengt. Durch/mit Einführung des chirurgischen Werkzeugs wird der Durchmesser des durch die Klemmarme radial begrenzten Hohlraums radial aufgeweitet und die Klemmarme werden in radialer Richtung federelastisch nach außen gedrückt. Durch die dabei sich aufbauende elastische Spannung der Klemmarme wird das eingeführte Werkzeug in dem Hohlraum des Hohlzylinders zwischen den Klemmarmen festgeklemmt. Hierbei sind die Klemmarme vorzugsweise so ausgebildet, dass die Kontaktfläche zwischen Klemmarmen und aufgenommenem Werkzeug möglichst gering ist (linien- und/oder punktförmig). Da die Oberfläche des Werkzeugs nur an den durch die Klemmarme definierten Punkten/Linien eine Kontaktstelle mit dem Hohlkörper der Aufnahmehülse bildet, befindet sich der Großteil der Oberfläche des in die Aufnahmehülse aufgenommene Anteils des Werkzeugs nicht in Kontakt mit dem Hohlkörper der Aufnahmehülse und ist somit von Reinigungsfluid umspülbar.

Es ist von besonderem Vorteil, dass durch Variieren des Durchmessers des Hohlzylinders und Anpassen der elastischen Verformbarkeit der Vorsprünge/Klemmarme sehr einfach verschiedene Aufnahmehülsen zur Aufnahme von chirurgischen Werkzeugen mit verschiedenen Schaftdurchmessern ausgebildet werden können.

In einer alternativen Ausführungsform sind die Klemmarme an der Innenseite der Aufnahmehülse nicht einstückig mit dem Hohlkörper der Aufnahmehülse ausgebildet, sondern separat beispielsweise aus Metall gefertigt und in den Hohlkörper der Aufnahmehülse eingelassen. Eine solche Fertigung der Klemmarme wirkt sich vorteilhaft auf die Lebensdauer und Robustheit der Aufnahmehülse aus.

Gemäß der Erfindung weist die Aufnahmehülse an ihrer Innenseite eine Anzahl starrer, axial verlaufender Vorsprünge (bzw. axial sich erstreckende Leitarme/Längsrippen) auf, die innerhalb des Hohlzylinders/Hohlkörpers der Aufnahmehülse einen Trichter bilden, der sich in Einführungsrichtung eines aufzunehmenden Werkzeugs verengt. Vorzugsweise bilden die Vorsprünge/Leitarme zusätzlich einen Auffangboden (Anschlag) in dem Hohlzylinder an einem anderen Endabschnitt (gegenüber der Einstecköffnung) der Aufnahmehülse, um ein zu tiefes Einführen eines Werkzeugs in die Aufnahmehülse zu verhindern. Durch die starren Leitarme wird ein Einführen eines Werkzeugs mit zu großem Schaftdurchmesser in die Aufnahmehülse verhindert, da die Leitarme im Gegensatz zu den Klemmarmen nicht/vernachlässigbar elastisch verformbar sind und somit klar einen Maximalschaftdurchmesser eines einführbaren Werkzeugs definieren. Eine erfindungsgemäße Aufnahmehülse kann nur eine Anzahl an Leitarmen, nur eine Anzahl an Klemmarmen oder aber auch eine Kombination von Leit- und Klemmarmen aufweisen.
Damit die Aufnahmehülse in eine Halterungsvorrichtung vorzugsweise bestehend aus zwei parallelbeabstandeten Lochplatten oder Matrices eingesetzt und darin fest verankert werden kann, weist die Aufnahmehülse in einer Ausführungsform der Erfindung an ihrer Außenseite (Mantelseite) mindestens einen elastisch verformbaren radial vorragenden Vorsprung oder Haltekeil auf. Wird die Aufnahmehülse beispielsweise in ein Loch der Halterungsvorrichtung (Lochmatrix/Lochplatte) eingesetzt, so wird der Vorsprung/Haltekeil durch die Begrenzung des Loches in radialer Richtung des Hohlzylinders nach innen gedrückt. Durch die elastische Spannung des Rückhaltearms wird die Aufnahmehülse in dem Loch der Lochmatrix dann in Position gehalten, indem sich bei vollständigem axialen Einschieben der Aufnahmehülse in die Halterungsvorrichtung (Lochplatte) der Vorsprung hinter das Loch der Halterungsvorrichtung (Lochplatte) anlegt und sich damit gegen die Halterungsvorrichtung (Lochmatrix/Lochplatte) axial abstützt.

In einer bevorzugten Ausführungsform der erfindungsgemäßen Aufnahmehülse umfasst diese zusätzlich einen umlaufenden ringförmigen Vorsprung (Kragen) entlang des Umfangs des Hohlzylinders im Bereich der Einstecköffnung sowie im Axialabstand zum Haltekeil, der bei eingesetztem Zustand der Aufnahmehülse in die Halterungsvorrichtung auf einer Lochmatrix vorzugsweise mit runden Löchern aufliegt und somit eine unerwünschte Bewegung der Aufnahmehülse in Einsetzrichtung und ein Hindurchfallen/Hindurchdrücken der Aufnahmehülse durch ein rundes Loch der Matrix verhindert. Damit wird die Halterungsvorrichtung/Lochmatrix zwischen dem Kragen und dem Haltekeil axial fixiert. Vorzugsweise ist der umlaufende Vorsprung an seiner der Einstecköffnung der Aufnahmehülse zugewandten Seite abgeflacht, sodass eine bedruckte Schablone formschlüssig auf der abgeflachten Seite des ringförmigen Vorsprungs aufgelegt werden kann. Weiterhin vorzugsweise weist der umlaufende ringförmige Vorsprung an seiner der Einstecköffnung der Aufnahmehülse abgewandten Seite Aussparungen/axiale Vorsprünge auf, wodurch die axiale Kontaktfläche zwischen Kragen und der Matrix der Halterungsvorrichtung verringert wird und etwaige Verschmutzungen, die sich zwischen Aufnahmehülse und Halterungsvorrichtung sammeln, effizient von Reinigungsfluid weggespült werden können.

In einer weiteren Ausführungsform der vorliegenden Erfindung weist die Aufnahmehülse an ihrem anderen axialen Ende (gegenüber der Einstecköffnung) eine Anzahl von axial sich erstreckende starren Vorsprüngen (Positionierzapfen) auf, die die Aufnahmehülse in Position halten, wenn diese in die Halterungsvorrichtung vollständig eingesetzt ist. Hierzu sei folgendes ausgeführt:
Eine erfindungsgemäße Halterungsvorrichtung umfasst beispielsweise eine erste Lochmatrix (Lochplatte) mit runden Löchern, in die die jeweils eine Aufnahmehülse eingesetzt ist und weiterhin parallel zu dieser Lochmatrix eine weitere, zweite Matrix (Lochplatte) beispielsweise mit eckigen Aussparungen/Löchern, deren Aussparungsflächen kleiner sind als die Flächen der runden Löcher der ersten Matrix, und die mit den runden Löchern der ersten Lochmatrix fluchten.

In diesem Ausführungsbeispiel sind die starren Positionierzapfen der Aufnahmehülse im Querschnitt eckig ausgebildet, sodass sie formschlüssig in die Ecken der eckigen Aussparungen der zweiten Matrix eingreifen und ein Verschieben/Verdrehen der Aufnahmehülse verhindern können. Wird eine zweite Matrix mit anders geformten Aussparungen/Löchern verwendet, so liegt es im Bereich der Erfindung, die Positionierzapfen der Aufnahmehülsen entsprechend anzupassen, sodass die Positionierzapfen formschlüssig in jeweils eine Aussparung/Loch der zweiten Matrix eingreifen.

Weiterhin umfasst die Aufnahmehülse in einer Ausführungsform an ihrem unteren Ende mindestens einen elastisch verformbaren, hakenförmigen sowie axial sich erstreckenden Vorsprung (Rastarm), der ähnlich den Positionierzapfen so ausgebildet ist, dass er formschlüssig beispielsweise in eine Ecke einer eckigen Aussparung der zweiten Matrix einer Halterungsvorrichtung eingreifen und/oder die vorzugsweise eckige Aussparung axial hintergreifen kann. Wird beispielsweise eine Aufnahmehülse in eine erfindungsgemäße Halterungsvorrichtung mit den zwei parallel angeordneten Matrices, wovon eine erste Matrix runde Löcher und eine zweite Matrix eckige Aussparungen/Löcher aufweist, die mit den runden Löchern der ersten Matrix fluchten, eingesetzt, so wird die Aufnahmehülse durch ein rundes Loch der ersten Matrix gedrückt, bis der Hohlzylinder an seinem Kragen auf der ersten Matrix zu liegen kommt und die erste Matrix zwischen sich und dem Haltekeil axial fixiert. Hierbei wird der mindestens eine elastisch verformbare Rastarm an dem anderen Ende des Hohlkörpers durch die Aussparung/eckiges Loch der zweiten Matrix gedrückt, bis der hakenförmige Vorsprung des Rastarms hinter der zweiten Matrix einrastet und somit eine Bewegung und ein Herausfallen der Aufnahmehülse entgegen der Einsetzrichtung verhindert. Da die Aussparrungsfläche der zweiten Matrix kleiner ist als die Lochfläche der ersten Matrix stützt sich die an die Lochfläche der ersten Matrix angepasste Aufnahmehülse mit ihrem Hohlkörper auf der zweiten Matrix axial auf und fixiert so die zweite Matrix zwischen dem Hohlkörper und dem axial vorragenden Rastarm. Damit ist auch der Axialabstand zwischen den beiden Matrices festgelegt.

D.h., weist eine Aufnahmehülse in einer Ausführungsform sowohl mindestens einen Rastarm, als auch einen Rückhaltearm (Haltekeil) auf, so legt die Aufnahmehülse den Abstand zwischen der ersten und zweiten Matrix fest, da die erste Matrix auf dem hakenförmigen Vorsprung des Rückhaltearms zu liegen kommt und die zweite Matrix durch den hakenförmigen Vorsprung des Rastarms in Position gehalten wird.

Ein weiterer Aspekt der vorliegenden Erfindung betrifft ein Werkzeugset zur Lagerung und Reinigung von chirurgischen Werkzeugen/Dentalwerkzeuge, das mindestens eine erfindungsgemäße Aufnahmehülse gemäß der vorstehenden Beschreibung ausweist. Weiterhin hat das Werkzeugset mehrere Außenwände und mindestens eine Innenwand (Boden und/oder einen Einsatz) als Halterungsvorrichtung, welche die Gestalt zweier parallelbeabstandeter Lochmatrices gemäß vorstehender Beschreibung hat, in die die mindestens eine erfindungsgemäße Aufnahmehülse eingesetzt/einsetzbar ist.

In ihrem Grundaufbau hat das Werkzeugset den Aufbau eines Kastens/Korbs mit einem Boden (Lochplatte oder Drahtgefecht), einem Deckel (Lochplatte oder Drahtgeflecht) vorzugsweise mit einem Griff und mehreren Außenwänden (Rahmen). Vorzugsweise weist der Deckel an mindestens zwei seiner Außenwände jeweils ein Zentrierblech auf, das ein passgenaues Aufsetzten des Deckels auf den Kasten erleichtert. Alternativ oder zusätzlich kann der Deckel an mindestens einer seiner Ecken einen Zentrierstift aufweisen, der in ein Zentrierloch in mindestens einer entsprechenden Ecke des Bodens/Seitenwand/Kastens eingreift und so ein passgenaues Aufsetzten des Deckels auf den Kasten gewährleistet. In geschlossenem Zustand wird der Deckel vorzugsweise durch einen Verschluss auf dem Kasten gehalten. Hierbei kann der Verschluss die Form eines federnden Bügels haben, der von außen auf die geschlossene Halterungsvorrichtung aufgeschoben werden kann und Deckel und Kasten/Boden der geschlossenen Halterungsvorrichtung umgreift. Alternativ kann ein federndes Blech oder ein Blech mit Gelenk verwendet werden, dass den Kasten/Boden der geschlossenen Halterungsvorrichtung umgreift und zudem in eine Aussparung in einer Seitenwand des Deckels eingreift.

Vorzugsweise bildet der Boden des Kasten/Korbs eine Halterungsvorrichtung gemäß der vorstehenden Beschreibung für die erfindungsgemäßen Aufnahmehülsen. Alternativ oder zusätzlich hierzu kann das Werkzeugset eine Innenwand/Einsatz/Zwischenetage aufweisen, die vorzugsweise die Gestalt zweier parallel zum Boden des Kastens angeordneter, sowie parallelbeabstandeter Bleche mit jeweils einer eingestanzten Lochmatrix gemäß der vorstehend beschriebenen Halterungsvorrichtung hat. In die Löcher dieser Lochmatrices können erfindungsgemäße Aufnahmehülsen wie oben beschrieben eingesetzt werden.

In einer Ausführungsform des erfindungsgemäßen Werkzeugsets sind die Außen-/Seitenwände und die mindestens eine Innenwand/die Halterungsvorrichtung von lichtem Aufbau, beispielsweise aus einem durchbrochenen Material, vorzugsweise aus einem Metallgitter oder -sieb oder einer Lochplatte gefertigt. Dies hat den Vorteil, dass Reinigungsfluide leicht in den Kasten und die Halterungsvorrichtung eindringen und die in den Aufnahmehülsen aufgenommenen Werkzeuge umspülen können. Hierbei muss die erfindungsgemäße Halterungsvorrichtung wie auch der Aufnahmekasten zur Reinigung nicht zerlegt oder die aufgenommen Werkzeuge entnommen werden, sondern die gesamte Halterungsvorrichtung und der Kasten können im bestückten Zustand gereinigt werden, ohne dass die Gründlichkeit der Reinigung der Halterungsvorrichtung/des Kastens oder der darin enthaltenen Werkzeuge beeinträchtigt wird. Da Kleinteile wie chirurgische Werkzeuge nicht mehr einzeln zur Reinigung aus den Aufnahmehülsen entnommen und separat gereinigt werden müssen und die Halterungsvorrichtung wie auch der Kasten zudem nicht mehr zerlegt und nach der Reinigung wieder zusammengesetzt werden muss, ist der für die Reinigung von Werkzeugen, Halterungsvorrichtung und Kasten benötigte Zeitaufwand stark verringert. Zudem ist das Reinigungsverfahren weniger anfällig für Anwenderfehler und schonender für die chirurgischen Werkzeuge, die während des gesamten Verfahrens in den Aufnahmehülsen verbleiben, wodurch Beschädigungen der Werkzeuge vermieden und deren Lebensdauer erhöht wird. Da weniger Handgriffe zur Reinigung der Werkzeuge, der erfindungsgemäßen Halterungsvorrichtung sowie des Kastens notwendig sind, wird der Erhalt der Sterilität der Werkzeuge zusätzlich erleichtert.

Die Verwendung von Metall bei der Fertigung der Halterungsvorrichtung und/oder des Kastens hat zudem den Vorteil, dass das Metall den hohen Temperaturen beispielsweise während der Sterilisation widerstehen kann, Verschmutzungen nur wenig an der Metalloberfläche haften bleiben und die Trocknung der Halterungsvorrichtung/des Kastens schneller und gründlicher erfolgt als bei einer Fertigung der Halterungsvorrichtung/des Kastens aus Kunststoff.

Neben den Aufnahmehülsen für chirurgische Werkzeuge kann die erfindungsgemäße Halterungsvorrichtung auch andere Lagerelemente für chirurgische Instrumente, beispielsweise Lagerelemente des aus dem Stand der Technik bekanten Aesculap-Instrumentenorganisationssytems (AIOS) aufweisen. Je nachdem, wie viele Werkzeuge in Aufnahmehülsen oder anderen Lagerelementen gelagert werden sollen, kann die Gestaltung der Halterungsvorrichtung entsprechend angepasst werden. In einem Ausführungsbeispiel weist 80% der Fläche der Halterungsvorrichtung eine Lochmatrix zum Einsetzten von Aufnahmehülsen auf, während die restlichen 20% der Fläche ein anders gestaltetes Raster zum Anbringen anderer Instrumente wie z. B. von AIOS-Lagerelementen bekannten Aufbaus aufweisen. Sind indessen so viele Werkzeuge und weitere Instrumente zu lagern, dass die Gesamtfläche der einen Halterungsvorrichtung in Form des entsprechend ausgebildeten Bodens des Werkzeugsets nicht ausreicht, so kann beispielsweise der zuerst als Halterungsvorrichtung vorgesehene Boden mit einem Raster zum Anbringen beispielsweise von AIOS- Lagerelementen bekannten Aufbaus ausgestattet werden und zusätzlich eine Zwischenetage parallel zu dem Boden eingezogen werden, welche dann die Halterungsvorrichtung vorstehend beschriebenen Aufbaus bildet, in die Aufnahmehülsen eingesetzt werden. Diese Zwischenetage besteht aus den oben beschriebenen ersten und zweiten Matrices, die beispielsweise jeweils runde Löcher und vorzugsweise eckige Aussparungen/Löcher aufweisen, in die Aufnahmehülsen gemäß der beschriebenen Konstruktion eingesetzt werden, wobei die Aufnahmehülsen durch etwaige Rückhaltearme, Positionierzapfen und Rastarme in Position gehalten werden und ggf. den Abstand der Matrices der Zwischenetage wie oben beschrieben festsetzen.

Eine solche Zwischenetage weist vorzugsweise mindestens einen Eckfuß auf, der in mindestens eine entsprechende Aussparung in einem inneren Vorsprung an einer Außenwand des Kastens eingreift und so ein passgenaues Aufsetzten der Zwischenetage auf dem Bodenteil des Kastens gewährleistet.

In einem Ausführungsbeispiel weist das Werkzeugset alternativ einen Kunststoffkasten auf, der eine erfindungsgemäße Halterungsvorrichtung in Form einer Zwischenetage beinhaltet. Die chirurgischen Werkzeuge können in diesem Fall in dem erfindungsgemäßen Werkzeugset gelagert werden, zur Reinigung der Werkzeuge verbleiben diese in der Halterungsvorrichtung und die Halterungsvorrichtung kann in bestücktem Zustand aus dem Werkzeugset/Kasten entnommen und gereinigt werden. Nach der Reinigung wird die bestückte Halterungsvorrichtung wieder in den Kunststoffkasten eingesetzt. Der Kunststoffkasten des Werkzeugsets ist hierbei von massivem Aufbau, was den Vorteil hat, dass die Sterilität der chirurgischen Werkzeuge für einen längeren Zeitraum gewährleistet werden kann und dennoch die Reinigung von chirurgischen Werkzeugen wesentlich vereinfacht ist.

Weitere Merkmale und Vorteile der vorliegenden Erfindung ergeben sich aus der folgenden beispielhaften Beschreibung einer besonders bevorzugten Ausführungsform der Erfindung anhand der Figuren. Dabei zeigt:
Fig.1 eine erfindungsgemäße Aufnahmehülse für ein chirurgisches Werkzeug, wobei Fig. 1a die Aufnahmehülse von ihrem ersten axialen Ende betrachtet und Fig. 1b die Aufnahmehülse von ihrem zweiten axialen Ende betrachtet zeigt,
Fig. 2 einen Längsschnitt durch eine Aufnahmehülse mit einem darin aufgenommen chirurgischen Werkzeug,
Fig. 3 eine Aufnahmehülse, die in einer Zwischenetage einer erfindungsgemäßen Halterungsvorrichtung eingesetzt ist, und
Fig. 4 eine Halterungsvorrichtung mit einer Zwischenetage für Aufnahmehülsen und einem Bodenteil zum Unterbringen von weiteren Instrumenten wie sogenannte AIOS-Lagerelemente.

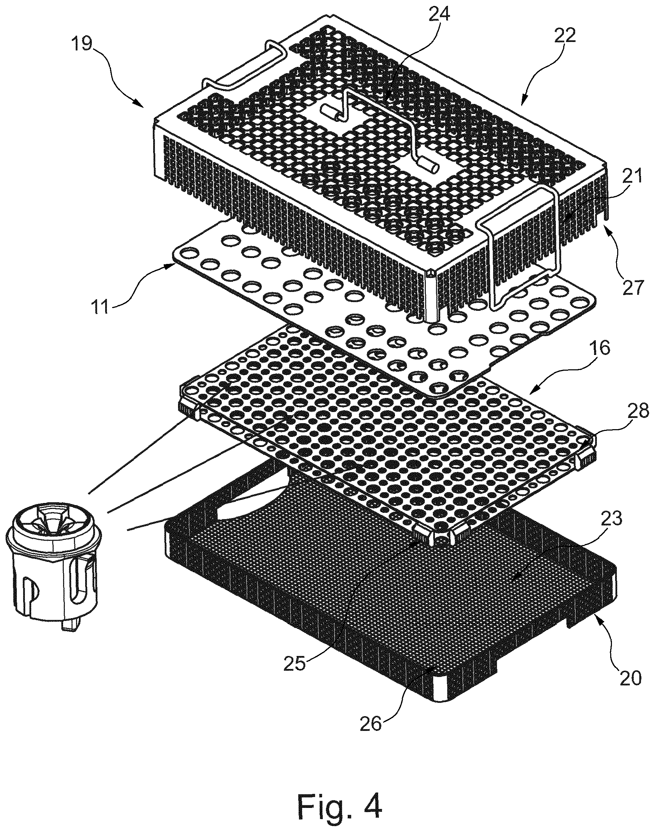

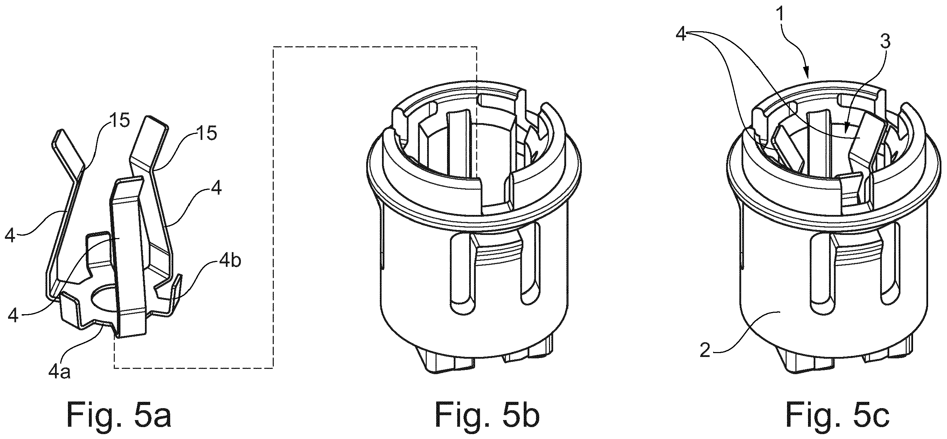

Zunächst wird mit Bezug auf die Fig. 4 ein erfindungsgemäßes Werkzeugset 19 mit einer Zwischenetage 16 für erfindungsgemäße Werkzeug-Aufnahmehülsen 1 und einem Bodenteil 20 zum Anbringen von AIOS- Lagerelementen beschrieben.

In seinem Grundaufbau hat das Werkzeugset 19 gemäß der Fig. 4 den Aufbau eines Kastens mit einem Bodenteil 20, der vorzugsweise als eine Halterungsvorrichtung für Aufnahmehülsen 1 konzipiert ist, einem Deckel 22 mit einem Griff 24 und mehreren Außenwänden (Rahmen). Weiterhin weist das Werkzeugset 19 einen Verschluss 21 in Form eines federnden Bügels auf, der von außen auf den geschlossenen Kasten aufgeschoben werden kann und Deckel 22 und Bodenteil 20 des geschlossenen Kastens umgreift.

Weiterhin beinhaltet das Werkzeugset 19 vorzugsweise eine Zwischenetage 16 als eine (weitere) Halterungsvorrichtung, in die erfindungsgemäße Aufnahmehülsen 1 wie nachfolgend noch beschrieben eingesetzt werden können. Auf die Zwischenetage 26 kann eine bedruckte Schablone 11 aufgelegt werden. Alternativ/zusätzlich weist der Bodenteil 20 des Werkzeugsets 19 ein Raster 23 zum Anbringen von AIOS-Lagerelementen auf.

Die Außenwände, der Deckel 22, der Bodenteil 20 und die optionale Zwischenetage 16 des Werkzeugsets 19 sind von lichtem Aufbau, d. h. aus einem durchbrochenen Material, hier aus einem Metallgitter oder Lochplatten gefertigt. Dies hat den Vorteil, dass Reinigungsfluide leicht in das Werkzeugset 19 eindringen und die in den Aufnahmehülsen 1 aufgenommenen Werkzeuge 14 umspülen können. Hierbei muss das erfindungsgemäße Werkzeugset 19 zur Reinigung nicht zerlegt oder die aufgenommen Werkzeuge 14 entnommen werden, sondern das gesamte Werkzeugset 19 kann im bestückten Zustand gereinigt werden, ohne dass die Gründlichkeit der Reinigung des Werkzeugsets 19 oder der darin enthaltenen Werkzeuge 14 beeinträchtigt wird.

Die Zwischenetage 16 weist vorzugsweise mindestens einen Eckfuss 25 auf, der in mindestens eine entsprechende Aussparung 26 in einem Vorsprung an einer Außenwand des Bodenteils 20 des Werkzeugsets 19 eingreift und so ein passgenaues Aufsetzten der Zwischenetage 16 auf den Bodenteil 20 des Werkzeugsets 19 gewährleistet und die Zwischenetage 16 in Position hält. Der Deckel 22 weist zudem an mindestens einer seiner Ecken einen Zentrierstift 27 auf, der in ein Loch 28 in mindestens einer entsprechenden Ecke der Zwischenetage 16 eingreift und so ein passgenaues Aufsetzten des Deckels 22 auf den Kasten gewährleistet.

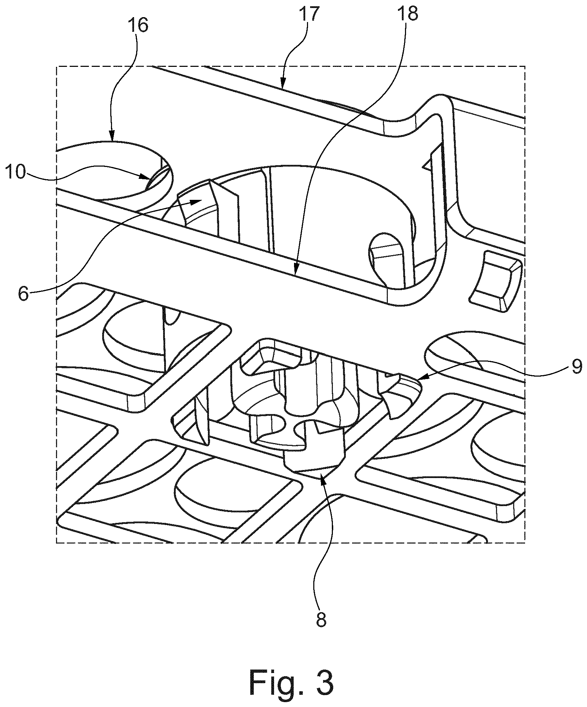

Die Haltevorrichtung für die Aufnahmehülsen 1 in Form des Bodenteils 20 und/oder der Zwischenetage 16 wird gemäß der Fig. 3 aus zwei parallelbeabstandeten Lochplatten 17, 18 gebildet, die vorzugsweise miteinander verbunden sind. An der einen Lochplatte 17 sind runde Löcher und an der anderen Lochplatte 18 sind vorzugsweise rechteckige Aussparrungen/Löcher ausgenommen, wobei die Löcher beider Lochplatten 17, 18 überlappend (co-axial) angeordnet sind, derart dass eine Aufnahmehülse 1 in zwei übereinander liegende Löcher beider Lochplatten 17, 18 eingeschoben werden kann.

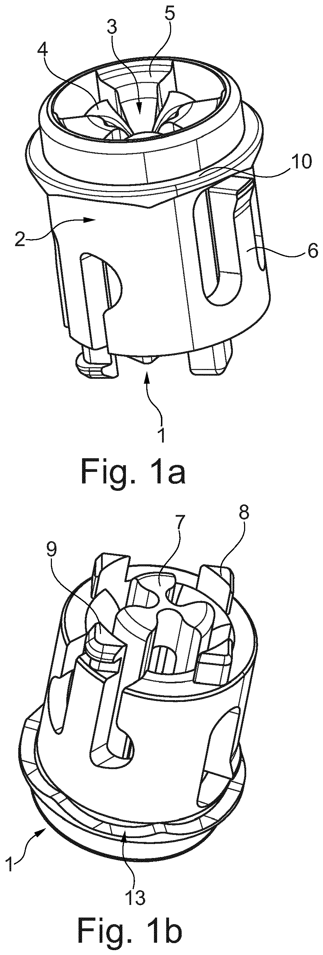

Figur 1a zeigt in Perspektivenansicht in Einführungsrichtung eines Werkzeugs 14 betrachtet, eine erfindungsgemäße Aufnahmehülse 1 mit einem Hohlkörper (bzw. Hohlzylinder) 2 von lichtem Aufbau (Filigranbauweise) mit einer axialen Werkzeug-Aufnahmeöffnung 3 an ihrem ersten axialen Ende, in die chirurgische Werkzeuge 14 eingeführt werden können. Die Aufnahmehülse 1 ist hierbei einstückig vorzugsweise im Spritzgussverfahren oder Rapid-Prototyping-Verfahren (Sinterverfahren) aus Kunststoff gefertigt. An ihrer Innenseite weist die Aufnahmehülse 1 drei oder mehr elastisch verformbare, axial sich erstreckende Klemmarme 4 auf, die innerhalb des Hohlzylinders 2 der Aufnahmehülse 1 vorzugsweise einen Trichter bilden, der sich in Einführungsrichtung eines aufzunehmenden Werkzeugs 14 verengt. Durch Einführung eines chirurgischen Werkzeugs, vorzugsweise Drehwerkzeugs (mit Werkzeugschaft) 14 wird der Durchmesser des durch die Klemmarme 4 radial begrenzten Hohlraums aufgeweitet und die Klemmarme 4 werden in radialer Richtung nach außen gedrückt. Durch die elastische Spannung der Klemmarme 4 wird das eingeführte Werkzeug 14 in dem Hohlraum des Hohlzylinders 2 zwischen den Klemmarmen 4 festgeklemmt. Hierbei sind die Klemmarme 4 vorzugsweise so ausgebildet, dass die Kontaktfläche 15 zwischen Klemmarmen 4 und aufgenommenem Werkzeug 14 möglichst gering (punkt-/linienförmig) ist. Außerdem halten die Klemmarme das Werkzeug in einem Radialabstand zum Hohlkörper 2.

Durch Variieren des Durchmessers des Hohlzylinders/Hohlkörpers 2 und Anpassen der elastischen Verformbarkeit der Klemmarme 4 können sehr einfach verschiedene Aufnahmehülsen 1 zur Aufnahme von chirurgischen Werkzeugen 14 mit verschiedenen Schaftdurchmessern ausgebildet werden. Alternativ können die Klemmarme 4 an der Innenseite der Aufnahmehülse 1 auch nicht einstückig mit dem Hohlkörper 2 der Aufnahmehülse 1 ausgebildet sein, sondern separat beispielsweise aus Metall gefertigt und in den Hohlkörper 2 der Aufnahmehülse 1 eingelassen sein. Eine solche Fertigung der Klemmarme 4 wirkt sich vorteilhaft auf die Lebensdauer und Robustheit der Aufnahmehülse 1 aus.

Weiterhin weist die Aufnahmehülse 1 an ihrer Innenseite drei oder mehr starre Leitarme/Längsrippen 5 auf, die innerhalb des Hohlzylinders 2 der Aufnahmehülse 1 in Umfangsrichtung gesehen zwischen den Klemmarmen 4 angeordnet sind und ebenfalls einen Trichter bilden, der sich in Einführungsrichtung eines aufzunehmenden Werkzeugs 14 verengt. Durch die starren Leitarme/Längsrippen 5 wird ein Einführen eines Werkzeugs 14 mit zu großem Schaftdurchmesser in die Aufnahmehülse 1 verhindert, da die Leitarme 5 im Gegensatz zu den Klemmarmen 4 nicht elastisch verformbar sind und somit klar einen Maximalschaftdurchmesser eines einführbaren Werkzeugs 14 definieren.

Damit die Aufnahmehülse 1 in die Halterungsvorrichtung bestehend aus den zwei Matrices 17, 18 eingesetzt und darin fest verankert werden kann, wie dies insbesondere in der Fig. 3 gezeigt wird, weist die Aufnahmehülse 1 weiterhin an ihrer Außenseite/Mantelfläche gemäß der Fig. 1a mindestens einen elastisch verformbaren Rückhaltearm/Haltekeil 6 auf. Wird die Aufnahmehülse 1 beispielsweise in ein rundes Loch der einen Lochmatrix 17 (siehe Fig. 3) der vorstehend beschrieben Halterungsvorrichtung eingesetzt, so wird der Rückhaltearm/Haltekeil 6 durch die radiale Begrenzung des Loches in radialer Richtung des Hohlzylinders 2 nach innen gedrückt. Durch die dabei sich aufbauende elastische Spannung des Rückhaltearms/Haltekeils 6 hintergreift der Rückhaltearm/Haltekeil 6 die Lochmatrix 17, sobald die Aufnahmehülse 1 in dem runden Loch der einen Lochmatrix 17 in axiale Position gelangt. Hierfür ist es in vorteilhafter Weise vorgesehen, dass das der Aufnahmeöffnung 3 zugewandte freie Ende des Rückhaltearms/Haltekeils 6 als radial nach Außen sich erstreckender Vorsprung/Klaue ausgebildet ist, der sich flächig hinter die eine Lochmatrix 17 axial anlegt.

Fig.1b zeigt in Perspektivenansicht entgegen der Einführungsrichtung eines Werkzeugs 14 betrachtet, die Aufnahmehülse 1 aus Figur 1a.

Aus dieser Ansicht ist erkennbar, wie die Leitarme/Längsrippen 5 einen Auffangboden/Endanschlag 7 in dem Hohlzylinder 2 der Aufnahmehülse 1 ausbilden, um ein zu tiefes Einführen eines Werkzeugs 14 in die Aufnahmehülse 1 zu verhindern. Im konkreten sind die Leitarme 5 an ihren axialen Enden gegenüber dem Werkzeug-Einführende durch einen Quersteg miteinander verbunden, der den Auffangboden/Endanschlag 7 darstellt.

Weiterhin weist die Aufnahmehülse 1 an ihrem dem Einführende gegenüberliegenden axialen zweiten Ende vorzugsweise drei starre Positionier-Längszapfen 8 auf, die die Aufnahmehülse 1 in Position halten, wenn diese in eine Halterungsvorrichtung gemäß der vorstehenden Beschreibung eingesetzt ist. Die Positionierzapfen 8 der Aufnahmehülse 1 sind im Querschnitt eckig/dreieckig ausgebildet, sodass sie formschlüssig in die Ecken der eckigen Aussparungen der anderen Matrix 18 gemäß der Fig. 3 eingreifen und ein Verschieben/Verdrehen der Aufnahmehülse 1 in der Haltevorrichtung verhindern können. Wird eine Matrix mit anders geformten Aussparungen/Löchern verwendet, so liegt es im Bereich der Erfindung, die Positionierzapfen 8 entsprechend geometrisch anzupassen, sodass die Positionierzapfen 8 formschlüssig in jeweils eine Aussparung/Loch der anderen Matrix 18 eingreifen können.

Die Aufnahmehülse 1 hat zudem an ihrem der Einstecköffnung 3 gegenüberliegenden axialen Ende mindestens einen elastisch verformbaren, hakenförmigen Rastarm 9, der sich ähnlich den Positionierzapfen 8 axial erstreckt und so ausgebildet ist, dass er formschlüssig beispielsweise in eine Ecke einer eckigen Aussparung der anderen Matrix 18 der Haltevorrichtung eingreifen und diese Matrix 18 axial hintergreifen kann, wie dies in der Fig. 3 gezeigt ist. Wird beispielsweise eine Aufnahmehülse 1 in eine erfindungsgemäße Halterungsvorrichtung (Boden 20 oder Zwischenetage 28) mit zwei parallel angeordneten Matrices 17, 18 eingesetzt, wovon die erste Matrix 17 runde Löcher und die parallel hierzu angeordnete zweite Matrix 18 eckige Aussparungen aufweist, die mit den runden Löchern der ersten Matrix 17 fluchten, so wird die Aufnahmehülse 1 durch ein Rundloch der ersten Matrix 17 gedrückt, bis der Hohlzylinder 2 auf der zweiten Matrix 18 zu liegen kommt, wie dies insbesondere in der Fig. 3 gezeigt ist. Hierbei wird der mindestens eine elastisch verformbare Rastarm 9 durch die Aussparung der zweiten Matrix 18 mit eckigen Löchern gedrückt, bis der hakenförmige Vorsprung des Rastarms 9 hinter der zweiten Matrix 18 einrastet und somit eine Bewegung und ein Herausfallen der Aufnahmehülse 1 entgegen der Einsetzrichtung verhindert.

Durch die Wirkung von Rastarm 9 und Rückhaltearm/Haltekeil 6 der Aufnahmehülse 1 legt bevorzugt die Aufnahmehülse 1 den parallelabstand zwischen der ersten und zweiten Matrix 17, 18 fest, da die erste Matrix 17 auf dem hakenförmigen Vorsprung des Rückhaltearms 6 zu liegen kommt und die zweite Matrix 18 durch den hakenförmigen Vorsprung des Rastarms 9 in Position gehalten wird.

Die Aufnahmehülse 1 hat weiterhin einen umlaufenden ringförmigen Vorsprung oder Kragen 10 entlang des Umfangs des Hohlzylinders 2 an ihrem einen axialen Endabschnitt im Bereich der Einstecköffnung 3, der bei eingesetztem Zustand der Aufnahmehülse 1 in der Halterungsvorrichtung auf der ersten Matrix 17 aufliegt und dabei die erste Matrix 17 zwischen sich und dem radialen Vorsprung des Rückhaltearms/Haltekeils 6 axial einklemmt und somit den Parallelabstand zwischen der ersten und zweiten Matrix 17, 18 gemäß der Fig. 3 fixiert. Somit wird auch eine unerwünschte Bewegung der Aufnahmehülse 1 in Einsetzrichtung und ein Hindurchfallen der Aufnahmehülse 1 durch ein Loch der ersten Matrix 17 verhindert. Der umlaufende Vorsprung 10 ist an seiner dem ersten Ende der Aufnahmehülse 1 zugewandten Seite abgeflacht, sodass eine bedruckte Schablone 11 formschlüssig auf der abgeflachten Seite des ringförmigen Vorsprungs 10 aufgelegt werden kann. Weiterhin weist der umlaufende ringförmige Vorsprung 10 an seiner dem zweiten Ende der Aufnahmehülse 1 zugewandten Seite Aussparungen 13 auf, wodurch die Kontaktfläche 15 zwischen umlaufendem Vorsprung 10 und erster Matrix 17 der Halterungsvorrichtung verringert wird und etwaige Verschmutzungen, die sich zwischen Aufnahmehülse 1 und Halterungsvorrichtung sammeln, effizient von Reinigungsfluid weggespült werden können.

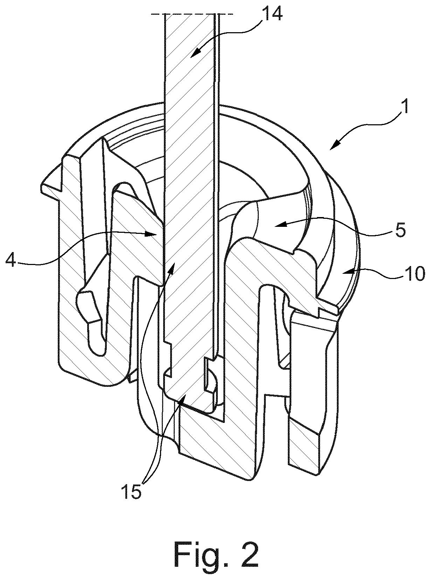

Figur 2 zeigt einen Querschnitt durch eine Aufnahmehülse 1 mit einem darin aufgenommenen chirurgischen Werkzeug 14.

Das Werkzeug 14 befindet sich demzufolge nur an den definierten Kontaktpunkten/- linien 15 im Klemmkontakt mit den Klemmarmen 4 und dem Auffangboden 7 der Aufnahmehülse 1. Hierbei sind die Klemmarme 4 vorzugsweise so ausgebildet, dass die Kontaktstellen 15 zwischen Klemmarmen 4 und aufgenommenem Werkzeug 14 möglichst klein sind. Da die Oberfläche des Werkzeugs 14 nur an den Kontaktstellen 15 an dem Hohlkörper 2 der Aufnahmehülse 1 kraftschlüssig anliegt, befindet sich der Großteil der Oberfläche des in die Aufnahmehülse 1 aufgenommene Anteils des Werkzeugs 14 nicht in Kontakt mit dem Hohlkörper 2 der Aufnahmehülse 1 und ist somit von Reinigungsfluid umspülbar.

Figur 3 zeigt schließlich eine Aufnahmehülse 1, die in der Zwischenetage 16 oder im Boden 20 des erfindungsgemäßen Werkzeugsets 19 eingesetzt ist. Die Zwischenetage 16 und/oder der Boden 20 besteht aus den zwei parallel zueinander angeordneten Blechen, wobei ein Blech die eingestanzte Lochmatrix 17 aufweist und das andere Blech die Matrix 18 aus eckigen Aussparungen aufweist, die mit den runden Löchern der Matrix 17 fluchten. Die Fläche der eckigen Aussparung der Matrix 18 ist hierbei kleiner als die Fläche des runden Lochs der Matrix 17.

Aus Figur 3 ist klar ersichtlich, wie die Positionierzapfen 8 der Aufnahmehülse 1 in die jeweils in eine Ecke einer eckigen Aussparung der Matrix 18 formschlüssig eingreifen und somit ein Verdrehen der eingesetzten Aufnahmehülse 1 verhindern. Zudem greift der Rastarm 9 formschlüssig in eine weitere Ecke der eckigen Aussparung der Matrix 18 ein, wobei sich der hakenförmige Vorsprung des Rastarms 9 unter das Blech der Matrix 18 schiebt und somit die Aufnahmehülse 1 in der Zwischenetage 16 bzw. im Bodenteil 20 verankert. Der radiale Vorsprung des Rückhaltearms 6 schiebt sich unter das Blech der Matrix 17 und dient zusätzlich der Verankerung der Aufnahmehülse 1 in der Zwischenetage 16 bzw. im Bodentel 20.

Der Abstand der beiden Matrices 17 und 18 ist, wie bereits angedeutet durch den Abstand der Vorsprünge des Rastarms 9 und des Rückhaltearms 6 definiert, auf denen jeweils die Matrix 17 und die Matrix 18 zu liegen kommen. Hierbei ist die Matrix 17 zwischen dem umlaufenden Vorsprung/Kragen 10 und dem Vorsprung des Rückhaltearms 6 in Position gehalten, während die Matrix 18 zwischen dem hakenförmigen Vorsprung des Rastarms 9 und dem Umfang des Hohlzylinders 2 fixiert ist.

## Patentansprüche

1. Aufnahmehülse eines Werkzeugsets zur lagernden Aufnahme chirurgischer Werkzeuge insbesondere Dental-Drehwerkzeuge, mit einem zylinderförmigen Hohlkörper (2), der an einer Stirnseite eine Einstecköffnung (3) für das Einstecken eines Werkzeugs ausbildet, wobei der zylinderförmige Hohlkörper (2) eine Anzahl von umfangsbeabstandeten inneren Klemmelementen (4) aufweist, die an ihren freien, vorzugsweise radial nach innen ragenden Enden punkt- oder linienförmige Eingriffsabschnitte haben, die zur kraft- und/oder formschlüssigen Kontaktierung eines eingesteckten Werkzeugs für das Halten des Werkzeugs innerhalb der Aufnahmehülse (1) im radialen Abstand zum zylinderförmigen Hohlkörper (2) angepasst sind, **dadurch charakterisiert, dass** an der Innenseite des Hohlkörpers (2) eine Anzahl von starren Vorsprüngen (5) angeordnet sind, die sich rippenförmig axial erstrecken und im Bereich der Einstecköffnung (3) trichterförmig abgeschrägt sind, wodurch sich eine Einsteckhilfe für das einzusteckende Werkzeug ergibt.

2. Aufnahmehülse nach Anspruch 1, **dadurch gekennzeichnet, dass** der zylinderförmige Hohlkörper (2) eine Anzahl von radialen Durchbrüchen als Einströmöffnungen für Reinigungsfluid aufweist.

3. Aufnahmehülse nach Anspruch 2, **dadurch gekennzeichnet, dass** die Klemmelemente (4) biegeelastische, vorzugsweise in Axialrichtung sich erstreckende Klemmarme sind, die scharnierartig am Hohlkörper (2) angeformt sind, wobei, vorzugsweise in Umfangsrichtung, zwischen den Klemmarmen (4) die radialen Durchbrüche im Hohlkörper (2) ausgenommen sind.

4. Aufnahmehülse nach einem der vorstehenden Ansprüche, **dadurch gekennzeichnet, dass** die Aufnahmehülse (1) einstückig, vorzugsweise durch ein Kunststoffspritzgussverfahren, ausgebildet ist.

5. Aufnahmehülse nach Anspruch 3, **dadurch gekennzeichnet, dass** die biegeelastischen Klemmarme (4) an der Innenseite des Hohlkörpers (2) aus Metall gefertigt und in den Hohlkörper (2) der Aufnahmehülse (1) eingelassen oder eingesetzt sind.

6. Aufnahmehülse nach einem der Ansprüche 1 bis 5, **dadurch gekennzeichnet, dass** die starren Vorsprünge (5) an einem der Einstecköffnung (3) gegenüberliegenden Endabschnitt der Aufnahmehülse (1) einen Auffangboden oder Anschlag (7) bilden, der ein zu tiefes Einführen des Werkzeugs in die Aufnahmehülse (1) verhindert.

7. Aufnahmehülse nach einem der vorhergehenden Ansprüche, **dadurch gekennzeichnet, dass** am zylinderförmigen Hohlkörper (2) eine Anzahl von umfangsbeabstandeten, vorzugsweise starren Positionszapfen (8) ausgebildet sind, die sich an dem der Einstecköffnung (3) gegenüberliegenden Endabschnitt des Hohlkörpers (2) über die Stirnseite des Aufnahmehülse (1) hinaus axial erstrecken.

8. Aufnahmehülse nach einem der vorhergehenden Ansprüche, **dadurch gekennzeichnet, dass** an dem der Einstecköffnung (3) gegenüberliegenden Endabschnitt des Hohlkörpers (2) mindestens ein weiterer biegeelastischer Rastvorsprung oder Rastarm (9) ausgeformt ist, der sich axial über die Stirnseite des Aufnahmehülse (1) hinaus axial erstreckt.

9. Aufnahmehülse nach einem der vorhergehenden Ansprüche, **dadurch gekennzeichnet, dass** der Hohlkörper (2) im axialen Bereich der Einstecköffnung (3) einen ringförmigen Vorsprung oder Kragen (13) aufweist, der als Axialanschlag für die Aufnahmehülse (1) dient.

10. Aufnahmehülse nach Anspruch 9, **dadurch gekennzeichnet, dass** der umlaufende ringförmige Vorsprung oder Kragen (13) auf seiner der Einstecköffnung (3) axial abgewandten Seite axial sich erstreckende Vorsprünge hat, die als punkt- oder linienförmige axiale Auflager oder Abstandshalter dienen.

11. Chirurgisches Werkzeugset zur Lagerung und Reinigung chirurgischer Werkzeuge, insbesondere Dental-Drehwerkzeuge, mit einem Aufnahmekasten bestehend aus einem Seitenrahmen, einem Bodenteil (20) und einem Zwischenetage (16), die in den Aufnahmekasten einsetzbar ist, **dadurch gekennzeichnet, dass** das Bodenteil (20) und/oder die Zwischenetage (16) in Form zweier parallelbeabstandeter, miteinander verbundener Lochplatten (17, 18) ausgebildet ist, die jeweils eine axial zueinander fluchtende Lochmatrix ausbilden und einem Deckel zum Verschließen des Aufnahmekastens, wobei in die Lochmatrices eine Anzahl von Aufnahmehülsen (1) gemäß einem der Ansprüche 1 bis 11 eingesetzt ist.

12. Chirurgisches Werkzeugset nach Anspruch 11, **dadurch gekennzeichnet, dass** zumindest der Kasten und vorzugsweise auch der Deckel aus einem durchbrochenen Material, vorzugsweise einem Metallgitter oder -sieb, ausgebildet sind.

13. Chirurgisches Werkzeugset nach Anspruch 11 oder 12, **dadurch gekennzeichnet, dass** die eine Lochplatte (17) runde Löcher und die andere Lochplatte (18) eckige Löcher hat, derart, dass die Aufnahmehülsen (1) an deren zylinderförmigen Hohlkörpern (2) in die runden Löcher bis maximal zum umlaufende Vorsprung oder Kragen (13) einschiebbar oder eingeschoben sind, wohingegen die starren Positionszapfen (8) in die Ecken der eckigen Löcher für ein Positionieren der Aufnahmehülsen (1) eingreifen.

14. Chirurgisches Werkzeugset nach Anspruch 13, **dadurch gekennzeichnet, dass** bei vollständig axial eingeschobener Aufnahmehülse (1) der wenigstens eine Rastarm (9) die Lochplatte mit eckigen Löchern hintergreift, wodurch die Aufnahmehülse (1) durch Zusammenwirken von Kragen (13) und Rastarm (9) axial in der Zwischenetage fixiert ist.

## Claims

1. A receiving sleeve of a tool set for the supporting mount of surgical tools, in particular dental rotary tools, comprising a cylindrical hollow body (2), having an end face provided with an insertion opening (3) for inserting a tool, wherein the cylindrical hollow body (2) comprises a number of circumferentially spaced inner clamping elements (4) which have punctiform or linear engagement sections which are provided at their free, preferably radially inward protruding ends and adapted for making a force-fit and/or form-fit contact with an inserted tool for holding the tool within the receiving sleeve (1) at a radial distance to the cylindrical hollow body (2), **characterized in that** the inner side of the hollow body (2) is provided with a number of rigid protrusions (5) which extend axially in rib-like fashion and are chamfered like a funnel in the area of the insertion opening (3), producing an insertion aid for a tool to be inserted.

2. Receiving sleeve according to claim 1, **characterized in that** the cylindrical hollow body (2) comprises a number of radial openings as inflow openings for a cleaning fluid.

3. Receiving sleeve according to claim 2, **characterized in that** the clamping elements (4) are flexible and elastic clamping arms which extend preferably in the axial direction and are formed on the hollow body (2) in the manner of a hinge, wherein the radial openings being cut out in the hollow body (2) preferably in the circumferential direction between the clamping arms (4).

4. Receiving sleeve according to any of the preceding claims, **characterized in that** the receiving sleeve (1) is formed in one piece, preferably by a plastic injection molding method.

5. Receiving sleeve according to claim 3, **characterized in that** the flexible and elastic clamping arms (4) situated on the inner side of the hollow body (2) are made of metal and embedded or inserted in the hollow body (2) of the receiving sleeve (1).

6. Receiving sleeve according to one of the claims 1 to 5, **characterized in that** the rigid protrusions (5) form a catching bottom or stop (7) on an end portion of the receiving sleeve (1) opposite the insertion opening (3), said catching bottom or stop preventing the tool from being inserted too deeply in the receiving sleeve (1).

7. Receiving sleeve according to any of the preceding claims, **characterized in that** a number of circumferentially spaced, preferably rigid positioning studs (8) is formed on the cylindrical hollow body (2) which extend axially beyond the end face of the receiving sleeve (1) on the end portion of the hollow body (2) opposite the insertion opening (3).

8. Receiving sleeve according to any of the preceding claims, **characterized in that** the end portion of the hollow body (2) opposite the insertion opening (3) is provided with at least one further flexible and elastic latching protrusion or latching arm (9) which extends axially beyond the end face of the receiving sleeve (1).

9. Receiving sleeve according to any of the preceding claims, **characterized in that** the hollow body (2) is provided in the axial area of the insertion opening (3) with an annular protrusion or collar (13) serving as an axial stop for the receiving sleeve (1).

10. Receiving sleeve according to claim 9, **characterized in that** the surrounding annular protrusion or collar (13) comprises axially extending protrusions on its side facing axially away from the insertion opening (3), which serve as punctiform or linear axial supports or spacers.

11. A surgical tool set for storing and cleaning surgical tools, in particular dental rotary tools, comprising a reception box consisting of a side frame, a bottom part (20) and an intermediate shelf (16) which can be placed in the reception box, **characterized in that** the bottom part (20) and/or the intermediate shelf (16) are realized in the form of two parallel and spaced, interconnected perforated plates (17, 18) each forming an axially aligned hole array, and a cover for closing the reception box, wherein a number of receiving sleeves (1) according to any of the claims 1 to 11 being inserted in the hole arrays.

12. Surgical tool set according to claim 11, **characterized in that** at least the box and preferably also the cover are formed from a perforated material, preferably a metal grid or screen.

13. Surgical tool set according to claim 11 or 12, **characterized in that** the one perforated plate (17) has round holes and the other perforated plate (18) has cornered holes such that the receiving sleeves (1) can be inserted or are inserted with their cylindrical hollow bodies (2) into the round holes as a maximum up to the surrounding protrusion or collar (13), whereas the rigid positioning studs (8) engage the corners of the cornered holes for positioning the receiving sleeves (1).

14. Surgical tool set according to claim 13, **characterized in that** the at least one latching arm (9) engages behind the perforated plate comprising the cornered holes when the receiving sleeve (1) is axially inserted to the full extent, whereby the receiving sleeve (1) is axially fixed in the intermediate shelf by the cooperation between the collar (13) and the latching arm (9).

## Revendications

1. Douille de réception d'un ensemble d'outils servant à recevoir par support des outils chirurgicaux, en particulier des outils rotatifs dentaires, avec un corps creux (2) en forme de cylindre, qui réalise au niveau d'un côté frontal une ouverture d'enfichage (3) pour l'enfichage d'un outil,
dans laquelle le corps creux (2) en forme de cylindre présente un nombre donné d'éléments de serrage (4) intérieurs espacés en périphérie, qui ont, au niveau de leurs extrémités libres, de préférence dépassant vers l'intérieur radialement, des sections de prise ponctuelles ou linéaires, qui sont adaptées pour établir un contact à force et/ou par complémentarité de forme avec un outil enfiché pour le maintien de l'outil à l'intérieur de la douille de réception (1) à distance radialement du corps creux (2) en forme de cylindre,
**caractérisée en ce que**
sont disposées au niveau du côté intérieur du corps creux (2) un nombre donné de parties faisant saillie (5) rigides, qui s'étendent de manière axiale de manière à présenter une forme de nervure et sont biseautées de manière à présenter une forme d'entonnoir dans la zone de l'ouverture d'enfichage (3), ce qui permet de donner lieu à une aide à l'enfichage pour l'outil à enficher.

2. Douille de réception selon la revendication 1, **caractérisée en ce que** le corps creux (2) en forme de cylindre présente un nombre donné d'ajours radiaux en tant qu'ouvertures de flux entrant pour un fluide de nettoyage.

3. Douille de réception selon la revendication 2, **caractérisée en ce que** les éléments de serrage (4) sont des bras de serrage élastiques à la flexion, de préférence s'étendant dans la direction axiale, qui sont formés à la manière d'une charnière au niveau du corps creux (2), dans laquelle les jours radiaux dans le corps creux (2) sont évidés de préférence dans la direction périphérique entre les bras de serrage (4).

4. Douille de réception selon l'une quelconque des revendications précédentes,
**caractérisée en ce que** la douille de réception (1) est réalisée d'un seul tenant, de préférence par un procédé de moulage par injection de matière synthétique.

5. Douille de réception selon la revendication 3, **caractérisée en ce que** les bras de serrage (4) élastiques à la flexion sont produits à partir de métal au niveau du côté intérieur du corps creux (2) et sont introduits ou insérés dans le corps creux (2) de la douille de réception (1).

6. Douille de réception selon l'une quelconque des revendications 1 à 5, **caractérisée en ce que** les parties faisant saillie (5) rigides forment au niveau d'une section d'extrémité, faisant face à l'ouverture d'enfichage (3), de la douille de réception (1), un fond formant réceptacle ou une butée (7), qui empêche une introduction trop profonde de l'outil dans la douille de réception (1).

7. Douille de réception selon l'une quelconque des revendications précédentes,
**caractérisée en ce que** sont réalisés au niveau du corps creux (2) en forme de cylindre un nombre donné de pivots de position (8) espacés en périphérie, de préférence rigides, qui s'étendent de manière axiale au niveau de la section d'extrémité, faisant face à l'ouverture d'enfichage (3), du corps creux (2) au-delà du côté frontal de la douille de réception (1).

8. Douille de réception selon l'une quelconque des revendications précédentes,
**caractérisée en ce qu'est** formée ou formé au niveau de la section d'extrémité, faisant face à l'ouverture d'enfichage (3), du corps creux (2), au moins une autre partie faisant saillie d'enclenchement ou un autre bras d'enclenchement (9) élastique à la flexion, qui s'étend de manière axiale au-delà du côté frontal de la douille de réception (1).

9. Douille de réception selon l'une quelconque des revendications précédentes,
**caractérisée en ce que** le corps creux (2) présente dans la zone axiale de l'ouverture d'enfichage (3) une partie faisant saillie ou un rebord (13) de forme annulaire, qui fait office de butée axiale pour la douille de réception (1).

10. Douille de réception selon la revendication 9, **caractérisée en ce que** la partie faisant saillie ou le rebord (13) de forme annulaire périphérique a des parties faisant saillie s'étendant de manière axiale sur son côté axialement opposé à l'ouverture d'enfichage (3), qui font office de supports ou d'écarteurs axiaux ponctuels ou linéaires.

11. Ensemble d'outils chirurgical servant à supporter et à nettoyer des outils chirurgicaux, en particulier des outils rotatifs dentaires, avec un boîtier de réception constitué d'un cadre latéral, d'une partie de fond (20) et d'un étage intermédiaire (16), qui peut être inséré dans le boîtier de réception, **caractérisé en ce que** la partie de fond (20) et/ou l'étage intermédiaire (16) sont réalisés sous la forme de deux plaques perforées (17, 18) espacées de manière parallèle, reliées l'une à l'autre, qui réalisent respectivement une matrice perforée en affleurement axial l'une par rapport à l'autre, et avec un couvercle servant à fermer le boîtier de réception, dans lequel un nombre donné de douilles de réception (1) selon l'une quelconque des revendications 1 à 11 est inséré dans les matrices perforées.

12. Ensemble d'outils chirurgical selon la revendication 11, **caractérisé en ce qu'**au moins le boîtier et de préférence également le couvercle sont réalisés à partir d'un matériau ajouré, de préférence d'une grille métallique ou d'un tamis métallique.

13. Ensemble d'outils chirurgical selon la revendication 11 ou 12, **caractérisé en ce qu'**une plaque perforée (17) a des trous ronds et l'autre plaque perforée (18) a des trous anguleux de telle manière que les douilles de réception (1) peuvent être enfilées ou sont enfilées au niveau de leurs corps creux (2) en forme de cylindre dans les trous ronds au maximum jusqu'à la partie faisant saillie ou le rebord (13) périphérique, alors que les pivots de position (8) rigides viennent en prise avec les angles des trous anguleux pour un positionnement des douilles de réception (1).

14. Ensemble d'outils chirurgical selon la revendication 13, **caractérisé en ce que** lorsque la douille de réception (1) est totalement enfilée axialement, l'au moins un bras d'enclenchement (9) vient en prise par l'arrière avec la plaque perforée comprenant des trous anguleux, ce qui a pour effet de fixer la douille de réception (1) du fait de la coopération du rebord (13) et du bras d'enclenchement (9) de manière axiale dans l'étage intermédiaire.
